**Europäisches Patentamt**

**European Patent Office**

**Office européen des brevets**

(19)

(11) Veröffentlichungsnummer : **0 499 799 B1**

(12)

# EUROPÄISCHE PATENTSCHRIFT

(45) Veröffentlichungstag der Patentschrift :
18.11.93 Patentblatt 93/46

(51) Int. Cl.$^5$ : **B01J 29/28**

(21) Anmeldenummer : **92100839.7**

(22) Anmeldetag : **20.01.92**

(54) **Katalysator zur Umwandlung technischer C8-Aromatenfraktionen.**

(30) Priorität : **20.02.91 DE 4105223**

(43) Veröffentlichungstag der Anmeldung :
**26.08.92 Patentblatt 92/35**

(45) Bekanntmachung des Hinweises auf die
Patenterteilung :
**18.11.93 Patentblatt 93/46**

(84) Benannte Vertragsstaaten :
**BE DE ES FR GB IT NL**

(56) Entgegenhaltungen :
**EP-A- 0 036 292**
**EP-A- 0 134 331**
**EP-A- 0 134 850**
**EP-A- 0 273 091**
**EP-A- 0 303 527**

(73) Patentinhaber : **LEUNA-WERKE AG**
**Am Haupttor**
**D-06236 Leuna (DE)**
Patentinhaber : **VAW Aluminium AG**
**Georg-von-Boeselager-Strasse 25**
**D-53117 Bonn (DE)**

(72) Erfinder : **John, Heino, Dr.**
**Brüderstrasse 15**
**O-4020 Halle (DE)**
Erfinder : **Neubauer, Hans-Dieter, Dr.**
**Sorbenweg 4**
**O-4200 Merseburg (DE)**
Erfinder : **Birke, Peter, Prof. Dr.**
**Block 620/4**
**O-4090 Halle (DE)**
Erfinder : **Berrouschot, Hans-Dieter**
**Weissenfelserstrasse 10**
**O-4851 Langendorf (DE)**
Erfinder : **Klempin, Jürgen, Dr.**
**Julian-Marchlewski-Ring 119**
**O-1330 Schwedt (DE)**
Erfinder : **Schreiber, Erhard**
**Fr.-Engels-Strasse 34**
**O-1330 Schwedt (DE)**

EP 0 499 799 B1

**Beschreibung**

Die Erfindung betrifft einen Katalysator zur Umwandlung von technischen $C_8$-Aromatenfraktionen in Gegenwart von Wasserstoff, die Xylene in Konzentrationen außerhalb des thermodynamischen Gleichgewichts sowie bis etwa 30 Masse-% Ethylbenzen enthalten.

Es ist aus DE-PS 2 442 241 bekannt, bei der Umwandlung von aromatischen $C_8$-Kohlenwasserstoffverbindungen einen Katalysator einzusetzen, der einen Zeolithen des Typs ZSM-5 enthält. Der Katalysator wird hergestellt, indem der Zeolith mittels eines Binders, wie Aluminiumoxid, geformt und gegebenenfalls durch Ionenaustausch mit einer Hydriermetallkomponente eines Metalls der VIII. Nebengruppe des Periodensystems der Elemente, wie Nickel, versehen wird.

Aus EP 243 684 sind Katalysatoren auf der Basis von Zeolithen mit Zwangsindices von 1 bis 12 bekannt, die ein Matrixmaterial, wie Aluminiumoxid, und 0,05 bis 10 Masse-% Hydrier-/Dehydrierkomponente, wie Platin, enthalten.

Katalysatoren auf Basis eines Pentasilzeolithen, der mit einem Binder, wie Aluminiumoxid, geformt und mit Platin als Hydriermetallkomponente versehen ist, werden u. a. in den EP 18 498, 38 141, 87 906, 151 351, 273 091 und 303 527 sowie den US-PS 4 873 387 und 4 899 012 beschrieben. Diese Katalysatoren sollen Xylene isomerisieren und Ethylbenzen dealkylieren, wobei eine hohe Ringerhaltungstendenz angestrebt wird. Das bedeutet, daß die Xylene selektiv ohne Verluste erhalten bleiben und Ethylbenzen aus dem $C_8$-Aromatenbereich weitgehend entfernt wird. Die selektive Umwandlung des Ethylbenzens zu Xylenen ist an diesem System kaum möglich, so daß bestenfalls die ursprünglich eingesetzte summarische Menge an Xylenen gewonnen werden kann.

Aufgabe der Erfindung ist es, einen Katalysator zu schaffen, der bei der Umwandlung technischer $C_8$-Aromatenfraktionen hochselektiv und hochaktiv ist sowie über längere Zeit stabil arbeitet und es gestattet, Xylene durch Isomerisierung so umzuwandeln, daß das thermodynamische Gleichgewicht zwischen den drei Isomeren eingestellt wird. Ethylbenzen soll gleichzeitig entweder

a) zu Benzen dealkyliert oder zu Benzen und $C_{10}$-Aromaten disproportioniert oder

b) zu p- und/oder o-Xylen isomerisiert werden.

Weiterhin sollen paraffinische Inhaltsstoffe durch Hydrospaltung in leicht abtrennbare Inhaltsstoffe umgewandelt werden.

Überraschend wurde ein Katalysator zur Umwandlung technischer $C_8$-Aromatenfraktionen in Gegenwart von Wasserstoff und basischen Stickstoffverbindungen gefunden, der 10 bis 70 Masse-% eines Pentasilzeolithen enthält und der vor der Einarbeitung in eine $Al_2O_3$-Matrix in einer Aluminiumnitratlösung suspendiert und mit Ammoniaklösung versetzt wurde, wobei die Zeolithkristalle mit 1 bis 5 Masse-% $Al_2O_3$, bezogen auf den Zeolith, als Pseudoböhmit umhüllt werden. Der Pentasilzeolith wird bei der Formgebung in eine Matrix von Aluminiumoxid eingearbeitet, die als Hydrierkomponente Platin enthält, und der Gehalt an Platin beträgt im Katalysator, bezogen auf die Gesamtzusammensetzung, 0,01 bis 0,1 Masse-%.

Vorzugsweise beträgt der Platingehalt im Katalysator, bezogen auf die wasser- und ammoniumfreie Basis, 0,01 bis 0,04 Masse-%. Die zeolithische Katalysatorkomponente besteht aus einem ZSM-5-ähnlichen Pentasilzeolith (MFI-Struktur). Das $SiO_2/Al_2O_3$-Molverhältnis des Zeoliths beträgt etwa 20 bis 55 : 1. Vorzugsweise wird ein Zeolith eingesetzt, der durch eine templatefreie Synthese erzeugt worden ist. Beispielsweise werden solche Zeolithe nach der DE-OS 3 919 098 erzeugt. In der DE-OS 4 022 140 sind die Eigenschaften eines auf diese Weise erzeugten Zeoliths beschrieben.

Nach der Synthese vorhandene Natriumionen müssen möglichst komplett auf einen Restgehalt von < 0,02 Masse % durch Ionenaustausch entfernt werden.

Der Zeolith wird folgendermaßen modifiziert:

Die Alkali- oder Ammoniumform des Zeoliths wird als Pulver in einer Aluminiumnitratlösung suspendiert (Flüssigkeits- zu Feststoff-Verhältnis in Masseteilen ca. 4 bis 6 : 1). Der Gehalt an Aluminiumionen in der Lösung ist so bemessen, daß nach Zusatz von Ammoniaklösung bis zu pH-Werten von 7 bis 8 auf den Zeolith 1 bis 5 % in Masseteilen, bezogen auf den Zeolith, als Pseudoböhmit aufgefällt werden, so daß die Zeolithkriställchen von einer Aluminiumoxidhydroxidschicht umhüllt sind. Dazu wird die Suspension unter Rühren auf 50 bis 70 °C erhitzt und Ammoniaklösung zugegeben, wobei der Pseudoböhmit ausfällt. Unter Rühren läßt man die Fällung ca. eine Stunde altern, dann wird filtriert und mit Wasser nachgewaschen. Falls der Natriumgehalt des Zeoliths noch zu hoch ist, muß mit einer Ammoniumsalzlösung (vorzugsweise Ammoniumnitrat bzw. -chlorid) so lange nachgewaschen werden, bis der gewünschte Wert sich einstellt. Dabei werden die Natriumionen durch Ammoniumionen ersetzt. Günstig für den Katalysator sind $Na_2O$-Gehalte von weniger als 0,01 % in Masseteilen, bezogen auf den bei 800 °C geglühten Zeolith. Sulfationen müssen möglichst vollständig ausgewaschen werden.

Der Zeolithfilterkuchen wird mit Pseudoböhmitpulver vermischt und mittels Presse zu Extrudaten geformt.

2

Der Teilchendurchmesser beträgt vorzugsweise 1 bis 3 mm. Ein besonderes Querschnittsprofil wird nicht benötigt. Die Formlinge werden bei 80 bis 120 °C getrocknet und bei 400 bis 700 °C geglüht, vorzugsweise bei 500 bis 600 °C. Dabei geht die Ammoniumform des Zeoliths unter Entweichen des Ammoniaks in die dekationisierte Form über.

Die erhaltenen Strangformlinge werden nach dem Glühen überstehend mit einer Lösung von Hexachloroplatinsäure getränkt. Nach ca. einer Stunde werden die $(PtCl_6)^{2-}$-Ionen vom Aluminiumoxid des Katalysators vollständig absorbiert. Ein Ionenaustausch am Zeolith findet dabei nicht statt, da die Anionen vom negativen Gitter des Zeoliths abgestoßen werden.

Der eingebaute Katalysator wird im Prozeß in Abhängigkeit von der aktuellen Aktivität in Gegenwart von basischen Stickstoffverbindungen betrieben, um seine Aktivität und Selektivität zu steuern und damit eine lange Laufzeit zu erreichen. Diese Methode der Steuerung wurde in der DD-PS 240 817 beschrieben und kann hier analog bzw. nach entsprechender Modifizierung in der Höhe des Ammoniakspiegels im Wasserstoffgas angewendet werden. Damit kann die durch die Art der Herstellung erreichte sehr hohe Katalysatoranfangsaktivität durch Blockierung der zeitweise nicht benötigten aziden Zentren gezielt auf das erforderliche Niveau gebracht werden. Durch die Belegung der am stärksten aziden Zentren werden weiterhin die unerwünschten Nebenreaktionen Disproportionierung und Spaltung der Xylene zurückgedrängt, so daß die Umwandlung optimal eingestellt werden kann. Während des Betriebs werden Aktivitätsverluste kontinuierlich durch Verringerung des Stickstoffverbindungsspiegels im Wasserstoffgas ausgeglichen. Damit kann man von einer hohen Katalysatorgrundaktivität ausgehen, ohne Selektivitätseinbußen in Kauf nehmen zu müssen. Verkokung und die im Zusammenhang stehende schnelle Desaktivierung werden auf diese Weise zurückgedrängt, und die Katalysatorlaufzeit wird wesentlich verlängert.

Der Prozeß wird durchgeführt, indem der erfindungsgemäße Katalysator im Wasserstoffstrom auf Temperaturen von 400 bis 600 °C aufgeheizt, mit basischen Stickstoffverbindungen, vorzugsweise Ammoniak, bis zum Durchbruch gesättigt, und bei Reaktionstemperaturen von etwa 400 bis 440 °C, Belastungen von 1 bis 10 v/vh, vorzugsweise von 3 bis 8 v/vh, Gas zu $C_8$-Aromatenverhältnissen von 150 bis 2000 : 1 Nl/l und Wasserstoffdrücken von 0,3 bis 1,5 MPa der Rohstoff zugeführt wird. Vorzugsweise arbeitet man bei geringen Gas- zu $C_8$-Aromatenverhältnissen. Zur Einstellung der Aktivität kann auch eine organische Stickstoffverbindung den Kohlenwasserstoffen zugemischt werden, wie z. B. Anilin oder Ethylendiamin. Die Stickstoffverbindungsgehalte im Rohstoff betragen bis ca. 200 ppm oder mehr, berechnet als Stickstoff, oder es wird ein bestimmter Ammoniakspiegel, z. B. 150 ppm, im Wasserstoffgas eingestellt.

Gegenüber den Katalysatoren gemäß Stand der Technik besitzt der erfindungsgemäße Katalysator eine sehr hohe Aktivität bei der Xylenisomerisierung. Unter technischen Bedingungen läßt er sich bei Belastungen von über 6 v/vh betreiben, wobei Xylenapproachwerte von deutlich über 90 % erreicht werden. Die Umwandlung des Ethylbenzens findet unter diesen Bedingungen ebenfalls noch zu einem hohen Grade statt. Der Approachwert für Ethylbenzen beträgt dabei mindestens noch 70 %. Bei geringerer Belastung werden Ethylbenzenapproachwerte von weit über 100 % erzielt, da dieses zu Benzen und Ethen gespalten, und damit aus dem $C_8$-Aromatenbereich entfernt wird. Trotz dieser hohen Umwandlungstiefe finden nur wenige Nebenreaktionen statt, so daß die Ausbeuten an Xylenen kaum niedriger sind als im Rohstoff bzw. bezogen auf den Rohstoff.

Aufgrund der hohen Selektivität der Reaktionen lagert sich während des Betriebs nur wenig "Koks" (wasserstoffarme Kohlenwasserstoffverbindungen) ab. Daher hat der Katalysator sehr lange Laufzeiten.

Ein weiterer, bedeutender Vorteil ist der niedrige Platingehalt des erfindungsgemäßen Katalysators. Dieser beträgt nur etwa ein Zehntel der sonst üblichen Platinmengen, die in den Katalysatoren gemäß Stand der Technik angewendet werden. Damit werden die Katalysatorkosten im Isomerisierungsverfahren deutlich vermindert.

Beispiel 1

Zwei Zeolithe mit einer MFI-Struktur, beide templatefrei synthetisiert, wurden als Ausgangsmaterialien verwendet.

Zeolith 1: Ein kommerzielles Produkt der Vereinigten Aluminiumwerke Bonn mit der Bezeichnung AlSi-Penta SN-27.
Eigenschaften: $SiO_2$ : $Al_2O_3$-Molverhältnis 27 : 1,
$Na_2O$-Gehalt 2,5 Masse-%, Teilchengröße 5 bis 50 µm.

Zeolith 2: Ein kommerzielles Produkt des VEB Chemiekombinat Bitterfeld mit der Bezeichnung Zeosorb HS-30
Eigenschaften: $SiO_2$ : $Al_2O_3$-Molverhältnis 30 : 1,
$Na_2O$-Gehalt 3,5 % in Masseteilen, Teilchengröße 15 bis 30 µm.

Die Zeolithe 1 und 2 wurden mit einer Ammoniumnitratlösung behandelt, um die Natriumionen gegen Ammoniumionen auszutauschen.

Bedingungen der Behandlung:

Die Zeolithe wurden als Pulver in einer 5%igen wäßrigen Ammoniumnitratlösung aufgeschlämmt, unter Rühren auf 70 °C erhitzt, mit Ammoniaklösung auf einen pH-Wert von 8,5 gebracht und danach filtriert und mit salzfreiem Wasser gewaschen. Der Filterkuchen wurde zur Herstellung von Vergleichskatalysatoren aufbewahrt.

Weiterhin wurden die Zeolithe 1 und 2 gemäß der vorliegenden Erfindung folgendermaßen modifiziert: 8 kg Zeolith wurden in einem 1000 Liter-Behälter unter Rühren in eine Lösung aus 400 l Wasser und 4 l Aluminiumnitratlösung (100 g/l $Al_2O_3$) eingetragen. Die Suspension wurde auf 70 °C hochgeheizt, mit Ammoniaklösung bis zum pH-Wert 8,0 neutralisiert, 30 Minuten nachgerührt bei 70 °C und über die Filterpressen filtriert. Danach wurde der Filterkuchen auf der Filterpresse mit 80 l Wasser gewaschen. Anschließend wurde eine 5%ige Ammoniaknitratlösung, die auf 70 °C aufgeheizt war, über die Filterpresse mit dem Zeolithfilterkuchen geschickt, um einen vollständigen Austausch der Natriumionen gegen Ammoniumionen zu erzielen. Danach wurde zum Nachwaschen alkalifreies Wasser bei 70 °C über die Filterpresse gedrückt, bis in der abfließenden Flüssigkeit keine Nitrationen mehr nachzuweisen waren.

Auf diese Weise wurde auf die Zeolithe ein $Al_2O_3$-Gehalt von 5 % in Masseanteilen aufgefällt. Das Aluminiumoxid befindet sich überwiegend auf den Zeolithkristallen als Aluminiumoxidhydrat in der Kristallstruktur des Pseudoböhmits, bzw. die Zeolithkriställchen werden durch Pseudoböhmit eingehüllt.

Weitere erfindungsgemäße Zeolithmodifizierungen mit $Al_2O_3$-Gehalten von 1,0, 2,0, 3,0 und 4,0 wurden mit HS-30 nach der eben beschriebenen Methode erzeugt.

In Tabelle 1 sind die hergestellten Zeolithkomponenten zusammengestellt.

## Tabelle 1: Modifizierung der Zeolithkomponenten

| Zeolithkomponente | Zeolithtyp | $Na_2O$ in %* | aufgefälltes $Al_2O_3$ in %* |
|---|---|---|---|
| 1 | HS-30 | 0,01 | 0,0 |
| 2 | SN-27 | 0,008 | 0,0 |
| 3 | HS-30 | 0,007 | 5,0 |
| 4 | SN-27 | 0,09 | 5,0 |
| 5 | HS-30 | 0,01 | 4,0 |
| 6 | HS-30 | 0,008 | 3,0 |
| 7 | HS-30 | 0,007 | 2,0 |
| 8 | HS-30 | 0,009 | 1,0 |

* in Masseteilen bezogen auf den bei 800 °C geglühten Zeolith. Die Angabe des $Al_2O_3$-Gehalts bezieht sich nur auf den aufgefällten Teil, nicht auf das Gitteraluminium des Zeoliths.

Jeweils 500 g der Zeolithkomponenten 1 bis 8 und 500 g getrocknetes und gemahlenes Aluminiumoxidhydrat vom Pseudoböhmittyp**, jeweils bezogen auf den Glührückstand bei 800 °C, wurden 5 bis 10 Minuten in einem Intensivmischer vermengt. Dann wurden 1 ml 50%ige Salpetersäure auf 100 ml verdünnt sowie 100 ml 5%ige Plastifikator-Lösung (ethoxylierte Zellulose) zugegeben, unter Intensivvermischung 5 Minuten peptisiert, ca. 400 ml Kondensat zugegeben und schließlich unter weiterer Intensivvermischung mit 2 ml Ammoniaklösung die Formungskonsistenz eingestellt.

Diese Mischungen wurden mittels einer Strangpresse durch Düsenplatten mit 1,5 mm Öffnungsdurchmes-

** gemahlen mittels Rohrschwingmühle auf eine Mahlfeinheit weniger als 1 % in Masseteilen Siebrückstand auf einem 10000er Maschensieb (kleiner/gleich 63 Mikrometer).

ser der Düsen zu kleinen Teilchen extrudiert, bei 120 °C zwei Stunden getrocknet und bei 600 °C fünf Stunden kalziniert.

Weiterhin wurden 200 g der Zeolithkomponente 4 und 800 g der Pseudoböhmitkomponente nach der gleichen, oben beschriebenen Art zu Teilchen geformt und thermisch nachbehandelt. Ein weiterer Ansatz aus 700 g der Zeolithkomponente 4 und 300 g der Pseudoböhmitkomponente wurde nach der gleichen Art geformt und thermisch nachbehandelt. Somit wurden die in Tabelle 2 zusammengestellten Formlingszusammensetzungen erhalten.

## Tabelle 2: Herstellung von Formlingen aus den modifizierten Zeolithkomponenten mit Pseudoböhmit als Binder

| Formlinge | Zeolithkomponente Nr. (Tabelle 1) % in Masseteilen | Pseudoböhmitkomponente % in Masseteilen |
|---|---|---|
| 1 | 1/50 | 50 |
| 2 | 2/50 | 50 |
| 3 | 3/50 | 50 |
| 4 | 4/50 | 50 |
| 5 | 5/50 | 50 |
| 6 | 6/50 | 50 |
| 7 | 7/50 | 50 |
| 8 | 8/50 | 50 |
| 9 | 4/20 | 80 |
| 10 | 4/70 | 30 |

Die Formlinge wurden mit einer Lösung von Hexachloroplatinsäure, die zusätzlich HCl enthielt, überstehend getränkt. Jeweils 400 g der Formlinge wurden in 1 Liter einer Lösung von Hexachloroplatinsäure, die 0,16 g Platin und zusätzlich 0,60 g HCl enthielt, getränkt, um Katalysatoren mit 0,04 Masse-% Platin herzustellen. Die Lösung wurde eine Stunde im Kreislauf durch die Formlingsschicht gepumt. Danach war das Platin aus der Lösung von den Formlingen absorbiert worden. Nach der Tränkung wurden die Formlinge bei 120 °C eine Stunde lang getrocknet und fünf Stunden bei 550 °C geglüht. Des weiteren wurden Katalysatoren mit anderen Platin- und Chlorkonzentrationen nach dem gleichen Verfahren durch Änderung der Platin- und Salzsäureangebote in der Lösung hergestellt. Eine Aufstellung der erzeugten Katalysatoren zeigt die folgende Tabelle 3.

Tabelle 3: Herstellung der Katalysatoren durch Tränkung der Formlinge mit Hexachloroplatinsäure + Salzsäure, trocknen und kalzinieren

| Katalysator | Formlingskomponente gemäß Tabelle 2 | Platin (jeweils in Masse-%) | Chlor |
|---|---|---|---|
| 1 | 1 | 0,04 | 0,16 |
| 2 | 2 | 0,04 | 0,16 |
| 3 | 3 | 0,04 | 0,16 |
| 4 | 4 | 0,04 | 0,16 |
| 5 | 5 | 0,04 | 0,16 |
| 6 | 6 | 0,04 | 0,16 |
| 7 | 7 | 0,04 | 0,16 |
| 8 | 8 | 0,04 | 0,16 |
| 9 | 9 | 0,04 | 0,16 |
| 10 | 10 | 0,04 | 0,16 |
| 11 | 4 | 0,01 | 0,04 |
| 12 | 4 | 0,08 | 0,32 |
| 13 | 4 | 0,15 | 0,60 |
| 14 | 4 | 0,30 | 1,20 |
| 15 | 1 | 0,08 | 0,32 |

Die Katalysatoren 1, 2, 13, 14 und 15 sind nicht erfindungsgemäß; die anderen sind erfindungsgemäß. Alle wurden mittels eines kleintechnischen Tests miteinander bezüglich ihrer Aktivität und Selektivität verglichen.

Die Katalysatoren wurden unzerkleinert in ein elektrisch beheizbares Reaktorrohr mit einem Innendurchmesser von etwa 21 mm eingebaut (100 ml Katalysatorvolumen). Der Katalysator wurde bei einem Gasdurchgang von 500 l Wasserstoff pro Stunde unter Normaldruck mit einer Geschwindigkeit von 20 K pro Stunde auf eine Temperatur von 500 °C aufgeheizt und eine Stunde bei dieser Temperatur belassen. Anschließend wurde die Arbeitstemperatur eingestellt (360 °C Anfangstemperatur) und der $H_2$-Druck auf 1,25 MPa gebracht. Unter diesen Bedingungen wurde ein technisches $C_8$-Aromatengemisch eingespritzt (300 ml /h). Die Zusammensetzung schwankte in relativ engen Grenzen. Ein Beispiel für die Zusammensetzung ist in der Tabelle 4 aufgeführt. Dem technischen $C_8$-Aromatengemisch wurden 150 ppm der basischen Stickstoffverbindung Anilin, berechnet als Stickstoff, zugegeben. Das Isomerisat von jeweils fünf Stunden Versuchsdauer wird zu je einer Bilanz zusammengefaßt und gaschromatographisch analysiert.

Tabelle 4: Rohstoff C$_8$-Aromatenfraktion

Die beiden Analysendaten zeigen die Konzentrationsschwankungen des eingesetzten Rohstoffs an.

| GC-Analyse Rohstoff | | 1 | 2 |
|---|---|---|---|
| Summe Nichtaromaten | : | 0,24 | 0,43 |
| Benzen | : | 0,39 | 0,00 |
| Toluen | : | 1,08 | 1,26 |
| Ethylbenzen | : | 23,44 | 25,88 |
| p-Xylen | : | 9,59 | 7,61 |
| m-Xylen | : | 48,43 | 49,34 |
| o-Xylen | : | 16,69 | 15,38 |
| p-MEB | : | 0,14 | 0,00 |
| m-MEB | : | 0,0 | 0,10 |
| o-MEB | : | 0,0 | 0,00 |
| 1.2.4-TMB | : | 0,0 | 0,00 |
| 1.2.3-TMB | : | 0,0 | 0,00 |
| C$_{10}$-Aromaten | : | 0,0 | 0,00 |
| Summe Xylene | : | 74,71 | 72,33 |
| Summe C$_8$-Aromaten | : | 98,15 | 98,21 |

MEB = Methylethylbenzen

TMB = Trimethylbenzen

Die Ziele der katalytischen Umwandlung bestehen darin,
- Ethylbenzen im C$_8$-Aromatengemisch weitgehend abzubauen (Approach größer/gleich 70 %) unter Bildung von Nicht-C$_8$-Aromaten (vorzugweise Benzen),
- das thermodynamische Gleichgewicht der Xylene auf annähernd 100 % einzustellen ($\geq$ 95 %) und
- die Xylene im Umwandlungsprodukt zu erhalten, d. h. möglichst keine Umwandlung dieser Stoffgruppe in Nicht-C$_8$-Aromaten zuzulassen (Xylenverluste bezogen auf den Ausgangsxylengehalt $\leq$ 0 %).

In Abhängigkeit von der Aktivität der Katalysatoren wird die Temperatur gesteigert, um diese Zielgröße einzustellen.

Berechnungsgrundlagen:

$$\text{Ethylbenzenapproach } (A_{EB}) \text{ in } \% = \frac{E_{EB} - E_{EI}}{E_{EB} - G_{EB}} \times 100 \%$$

Dabei sind:

$E_{EB}$ - Ethylbenzenkonzentration im Rohstoff, bezogen auf C$_8$-Aromaten in Ma.-%

$E_{EI}$ - Ethylbenzenkonzentration im Isomerisat, bezogen auf C$_8$-Aromaten in Ma.-%

$G_{EB}$ - Gleichgewichtskonzentration für Ethylbenzen ($G_{EB}$ = 8,5 %)

$$\text{p - Xylenapproach } (A_{p\text{ - }x}) \text{ in } \% = \frac{X_I - X_E}{G_X - X_E} \times 100 \%$$

Dabei sind:

$X_E$-p-Xylenkonzentration im Rohstoff, bezogen auf Xylene in Masse-%

$X_I$-p-Xylenkonzentration im Isomerisat, bezogen auf Xylene in Masse-%

$G_x$-Gleichgewichtskonzentration für p-Xylen ($G_x$ = 24 %)

Xylenverluste: Differenz zwischen der Summe an Xylenen im Rohstoff und der Summe der Xylene im Reaktionsprodukt jeweils in Masseanteilen.

Die Ergebnisse sind in Tabelle 5 zusammengefaßt.

## Tabelle 5: Ergebnisse des katalytischen Tests

Reaktionsbedingungen:

N-Gehalt im Rohstoff in ppm: 150 (als Anilin)

| | |
|---|---|
| GPV | : 500 : 1 Nl/l |
| Belastung | : 3 v/vh |
| Temperatur | : 430 °C |
| $H_2$-Druck | : 1,2 MPa |

| Katalysator-Nr. | $A_{EB}$ (%) | $A_{p-x}$ (%) | XV (%) |
|---|---|---|---|
| 1 | 62,0 | 89,0 | 1,0 |
| 2 | 67,1 | 88,8 | 1,5 |
| 3 | 90,1 | 96,5 | - 0,9 |
| 4 | 92,8 | 97,7 | - 1,1 |
| 5 | 88,5 | 96,0 | - 1,5 |
| 6 | 86,5 | 95,6 | - 0,8 |
| 7 | 82,0 | 95,2 | - 0,6 |
| 8 | 78,0 | 95,0 | 0,0 |
| 9 | 75,2 | 95,6 | - 1,0 |
| 10 | 92,5 | 97,0 | - 0,5 |
| 11 | 91,5 | 96,0 | - 1,1 |
| 12 | 85,2 | 99,5 | - 0,07 |
| 13 | 105,0 | 92,0 | 2,5 |
| 14 | 121,0 | 90,0 | 6,2 |
| 15 | 76,0 | 91,0 | 3,2 |

$A_{EB}$ (%) = Ethylbenzenapproach

$A_{p-x}$ (%) = p-Xylenapproach

XV (%) = Xylenverluste, bezogen auf die Xylenmenge im Rohstoff in % Masseanteile

Mit den erfindungsgemäßen Katalysatoren 3 bis 12 kann man unter den günstigen technischen Bedingun-

EP 0 499 799 B1

gen grundsätzlich Ethylbenzenapproachwerte von über 70 % erreichen, während bei den nicht erfindungsgemäßen Katalysatoren 1 und 2 diese Werte tiefer liegen. Mit den Katalysatoren 13, 14 und 15 werden zwar hohe Ethylbenzenapproachwerte erzielt, aber die Xylenverluste sind deutlich höher als bei den erfindungsgemäßen Katalysatoren. Die p-Xylenapproachwerte sind bei den erfindungsgemäßen Katalysatoren über 95 %, während die nicht erfindungsgemäßen Katalysatoren nicht eine so hohe Einstellung des thermodynamischen Gleichgewichts erlauben. Damit ist die Überlegenheit der erfindungsgemäßen Katalysatoren gegenüber denen gemäß Stand der Technik erwiesen.

Beispiel 2

Ein Katalysator gemäß Stand der Technik wurde folgendermaßen hergestellt:
Der Pentasilzeolith Zeosorb HS-30 wurde in die Ammoniumform durch Ionenaustausch mit einer 5%igen Ammoniumnitratlösung überführt (Rest-$Na_2O$-Gehalt $\leqq$ 0,01 % in Masseteilen,bezogen auf den bei 800 °C kalzinierten Zeolith). Der so erhaltene ammoniumausgetauschte Zeolith wurde in einer Eisen(III)nitratlösung aufgeschlämmt, unter Rühren auf 90 °C erwärmt und durch Zugabe von Ammoniaklösung auf einen pH-Werte von 8,0 gebracht. Anschließend wurde der mit Eisenhydroxid modifizierte Zeolith filtriert, mit salzfreiem Wasser gewaschen, getrocknet und auf eine Mahlfeinheit von weniger als 1 % in Masseteilen Durchgang durch ein 10000er Maschensieb ($\leqq$ 0,063 mm) gemahlen. Der so erhaltene modifizierte Zeolith wurde mit 30 % in Masseteilen Pseudoböhmitpulver, jeweils bezogen auf die bei 800 °C geglühten Substanzen, mit Wasser, etwas verdünnter Salpetersäure und wenig Ammoniaklösung intensiv vermischt, zu Formlingen mit einem Durchmesser von etwa 1,5 mm extrudiert, die bei 120 °C getrocknet und bei 550 °C geglüht wurden. Dieser so erhaltene, nicht erfindungsgemäße Vergleichskatalysator 16 wurde mit dem in Beispiel 1 beschriebenen erfindungsgemäßen Katalysator 4 unter gleichen Reaktionsbedingungen wie in Beispiel 1 verglichen.

Tabelle 6:   Ergebnis des katalytischen Tests mit
             Katalysator 16

| Katalysator-Nr. | 16 |
|---|---|
| $A_{EB}$ (%) | 45,0 |
| $A_{p-x}$ (%) | 90,0 |
| XV (%) | 0,0 |

Daraus wird ersichtlich, daß der Vergleichskatalysator 16 unter den angestrebten technisch günstigen Reaktionsbedingungen keine ausreichende Leistung erreicht.
Die Katalysatoren 4 (erfindungsgemäß) und 16 wurden in einem Dauertest 500 Stunden unter den Reaktionsbedingungen, wie in Beispiel 1 beschrieben, geprüft. Während der Katalysator 16 nach dieser Zeit nur noch ein Ethylbenzenapproach von 23 % einstellte, zeigte der erfindungsgemäße Katalysator 4 innerhalb von 450 Stunden so gut wie keinen Abfall der Aktivität und Selektivität. Damit wurde nachgewiesen, daß mit dem Katalysator gemäß vorliegender Erfindung sehr lange Laufzeiten erreicht werden können.

**Patentansprüche**

1.  Katalysator zur Umwandlung technischer $C_8$-Aromatenfraktionen in Gegenwart von Wasserstoff und basischen Stickstoffverbindungen auf der Basis eines Zeolithen, der in eine $Al_2O_3$-Matrix eingearbeitet ist, gekennzeichnet dadurch, daß er aus Zeolithkristallen, die mit 1 bis 5 Masse-% $Al_2O_3$ umhüllt sind, und einer $Al_2O_3$-Matrix, die 0,01 bis 0,1 Masse-% Platin, bezogen auf die Gesamtzusammensetzung, enthält,besteht.

2.  Katalysator nach Anspruch 1, gekennzeichnet dadurch, daß der Zeolithgehalt 10 bis 70 Masse-% beträgt.

3.  Katalysator nach Anspruch 1 oder 2, gekennzeichnet dadurch, daß der Zeolith ein Pentasilzeolith ist.

4.  Katalysator nach Anspruch 1 oder 3, gekennzeichnet dadurch, daß der Zeolith ein Pentasilzeolith mit MFI-

9

Struktur ist.

5. Katalysator nach einem der Ansprüche 1 bis 4, gekennzeichnet dadurch, daß der Platingehalt im Katalysator 0,01 bis 0,04 Masse-% beträgt.

## Claims

1. Catalyst for conversion of industrial $C_8$-aromatics fractions in the presence of Hydrogen und basic Nitrogen compounds, using a zeolite embedded in an $Al_2O_3$-matrix, characterized in that the latter is composed of crystalline zeolite wrapped-up in $Al_2O_3$, amounting to a weightpercentage of 1 to 5 %, and an $Al_2O_3$-matrix, containing 0.01 to 0.1 % by weight of platinum, always relating to the total weight.

2. Catalyst according to Claim No. 1, characterized in that the content of zeolite is 10 to 70 % by weight.

3. Catalyst according to Claim No. 1 or Claim No. 2, characterized in that pentasil zeolite is used as zeolitic compound.

4. Catalyst according to Claim No. 1 or Claim No. 3, characterized in that pentasil zeolite with MFI structure is used as zeolitic compound.

5. Catalyst according to Claim No. 1 through to and including Claim No. 4, characterized in that the content of platinum in the catalyst is 0.01 to 0.04 % by weight.

## Revendications

1. Catalyseur pour la transformation des fractions d'aromatiques $C_8$ techniques en présence de l'hydrogène et des composés d'azote basiques sur la base d'une zéolithe qui est incorporée dans une matrice d'$Al_2O_3$, caractérisé en ce qu'il consiste en cristaux de zéolithes enveloppés de 1-5 %en masse d'$Al_2O_3$, et en une matrice d'$Al_2O_3$, contenant 0,01-0,1% en masse de platine, rapporté à la composition totale.

2. Catalyseur selon la revendication 1, caractérisé en ce que la teneur en zéolithe s'élève à 10-70% en masse.

3. Catalyseur selon la revendication 1 ou 2, caractérisé en ce que la zéolithe est une pentasil zéolithe.

4. Catalyseur selon la revendication 1 ou 3, caractérisé en ce que la zéolithe est une pentasil zéolithe avec une structure MFI.

5. Catalyseur selon une des revendications 1 jusqu'au 4, caractérisé en ce que la teneur en platine dans le catalyseur s'élève à 0,01-0,04 % en masse.